# EUROPEAN PATENT APPLICATION

(11) **EP 3 502 124 A1**
(43) Date of publication of application: **26.06.2019**
(21) Application number: 16913261.0
(22) Date of filing: 19.08.2016
(51) Int. Cl.: C07K 7/62, A61K 38/12, A61P 31/04

(54) **CRYSTAL OF SULFATE OF POLYMYXIN B1, B2, OR MIXTURE THEREOF, AND MANUFACTURING METHOD OF SAME**

(71) Applicant: Hubei Ruihaoanke Pharmaceutical Technology Development Co., Ltd., Wuhan, Hubei 430071 (CN)
(72) Inventor: LI, Xiaobing, Wuhan Hubei 430071 (CN); LUO, Fen, Wuhan Hubei 430071 (CN); WANG, Zengxia, Wuhan Hubei 430071 (CN); XU, Yanwei, Wuhan Hubei 430071 (CN); LI, Changhong, Wuhan Hubei 430071 (CN)
(74) Representative: Uexküll & Stolberg
(86) International application number: PCT/CN2016/096061
(87) International publication number: WO 2018/032505

(57) **Abstract**

The present invention provides an anhydrous crystal of polymyxin B1 sulfate, polymyxin B2 sulfate or a mixture thereof and a preparation method thereof. The preparation method comprises using an organic solvent to precipitate a solid from a saturated solution of polymyxin B1 sulfate, polymyxin B2 sulfate or a mixture thereof, drying it under vacuum to obtain an anhydrous crystal of polymyxin B1 sulfate, polymyxin B2 sulfate or a mixture thereof.

## Description

### Technical field

The present invention belongs to the technical field of pharmacy, and in particular relates to a crystal of polymyxin B1 sulfate, polymyxin B2 sulfate or a mixture thereof and a preparation method thereof.

### Background Art

Polymyxin B is an alkaline cyclic polypeptide antibiotic produced by *bacillus polymyxa* and composed of various amino acids and fatty acids. Polymyxin B product is a multi-component compound, including polymyxin B1, B2, B3, B1-I (it is required that the sum of these four components ≥80.0% in the European Pharmacopoeia), and its sulfate is commonly used, which is a white or off-white powder with hygroscopicity. Polymyxin B sulfate has a strong killing effect on Gram-negative bacteria, and in particular, it renders high *in vitro* sensitivity to NDM-1 bacteria (super bacteria), and thus has attracted much attention. Since the two components, polymyxin B3 and B1-I, are nephrotoxic, a mixture containing the four components is mainly for external use clinically. The two components, B1 and B2, have low side effects and can be used for injection. High levels of polymyxin B1 and B2 crystals have higher product quality and can be more used for injection.

As for the refining of polymyxin B sulfate product, it is usually carried out by a spray drying method (patent application CN201210519331.7) or a lyophilization method (patent application CN201510775580.6) due to its difficulty to crystallize. The current patent about polymyxin B crystal includes polymyxin B1 dihydrate crystal disclosed in the patent application CN201210379231.9, which explicitly claims a compound having a molecular formula of C₅₆H₉₈N₁₆O₁₃·2H₂O, which is not a sulfate. The polymyxin B1 dihydrate crystal of the patent is obtained by precipitation using a mixture of acetone and diethyl ether. Diethyl ether is extremely volatile and easily to be oxidized in air and causes an explosion, which is not suitable for industrial production. In addition, the products on the market are all mixtures of polymyxin B sulfate. Regarding the preparation of polymyxin B1 sulfate monomer, the patent application filed by the present applicant has been granted (Patent No. ZL201110390624.5), in which the polymyxin B1 sulfate has a purity of 99.5%, and its solid is obtained by spray drying method. However, the polymyxin B sulfate prepared by the current spray drying method is difficult to form a crystal form, and the product is very easy to agglomerate, which brings inconvenience to production and research, and also affects the quality and efficacy of the drug.

### Description of the Invention

In view of the problems existing in the prior art, the present invention provides a crystal of polymyxin B1 sulfate, polymyxin B2 sulfate or a mixture thereof and a preparation method thereof, wherein polymyxin B1 sulfate, polymyxin B2 sulfate or a mixture thereof can be prepared by the method described in the patent ZL201110390624.5.

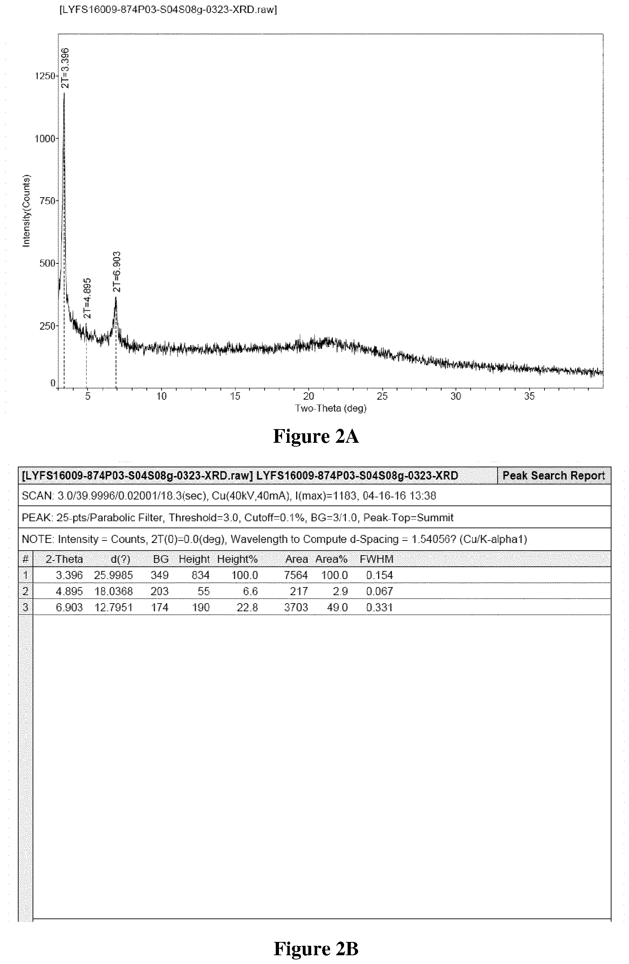

The present invention provides an anhydrous crystal 1 of polymyxin B1 sulfate, said anhydrous crystal 1 has an X-ray powder diffraction pattern having diffraction peaks at 3.396, 4.895 and 6.903 expressed by 2θ degree using Cu-Ka radiation; preferably, said anhydrous crystal 1 has an X-ray powder diffraction pattern expressed by 2θ degree using Cu-Ka radiation as shown in Figure 2A.

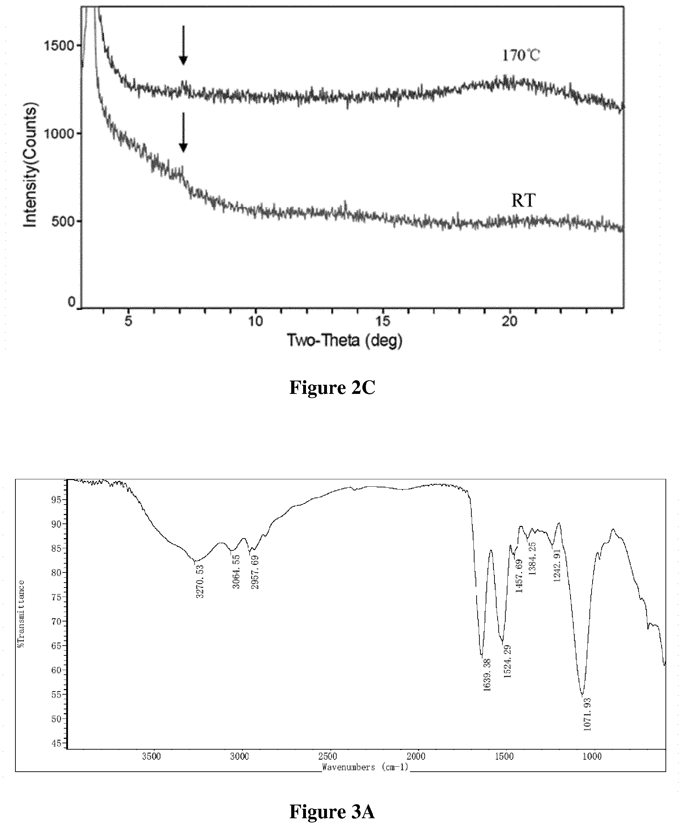

Preferably, said anhydrous crystal 1 has an infrared absorption spectrum having characteristic bands at 1071.93 cm⁻¹, 1242.91 cm⁻¹, 1384.25 cm⁻¹, 1457.69 cm⁻¹, 1524.29 cm⁻¹, 1639.38 cm⁻¹, 2957.69 cm⁻¹, 3064.55 cm⁻¹ and 3270.53 cm⁻¹ as measured by KBr tableting method; and more preferably, said anhydrous crystal 1 has an infrared absorption spectrum as measured by KBr tableting method as shown in Figure 3A.

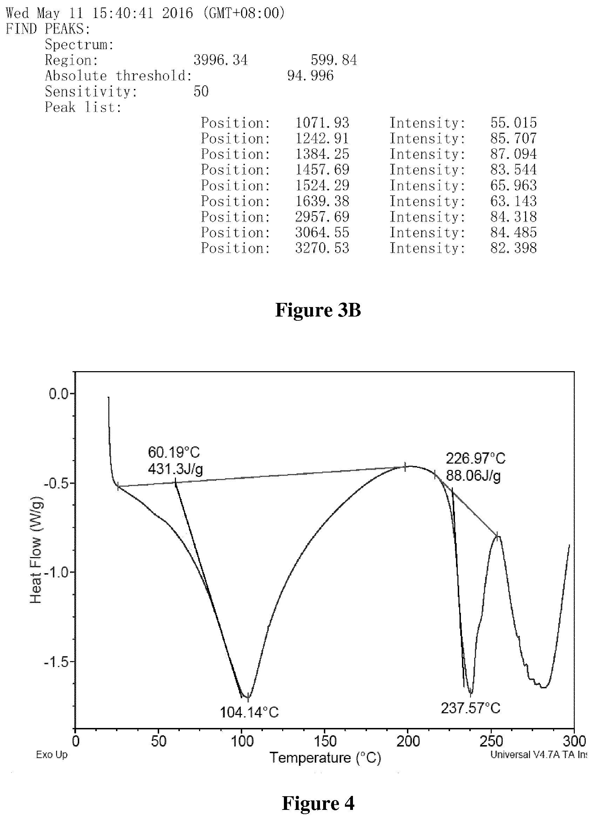

More preferably, said anhydrous crystal 1 has a melting point of 226.97 °C, and has a differential scanning calorimetry pattern as shown in Figure 4.

The present inventor conducted a more detailed analysis on anhydrous crystal 1, in which said anhydrous crystal 1 exhibits a dynamic moisture adsorption analysis spectrum as shown in Figure 5, a thermogravimetric analysis spectrum as shown in Figure 6, and an isotherm diagram as shown in Figure 7.

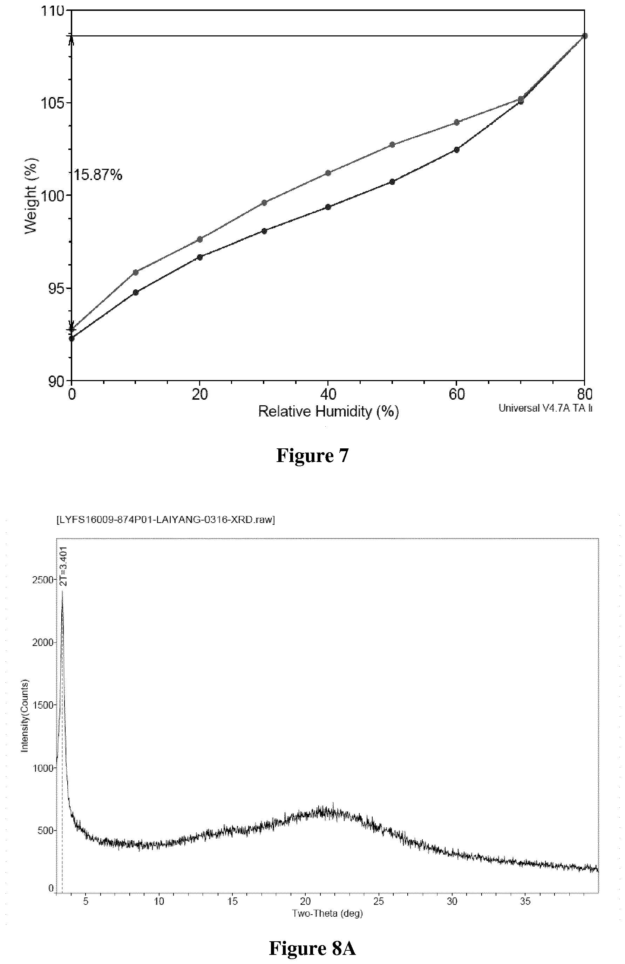

The present invention also provides an anhydrous crystal A of polymyxin B1 sulfate, said anhydrous crystal A has an X-ray powder diffraction pattern having a diffraction peak at 3.401 expressed by 2θ degree using Cu-Ka radiation; and preferably, said anhydrous crystal A has an X-ray powder diffraction pattern expressed by 2θ degree using Cu-Ka radiation as shown in Figure 8A.

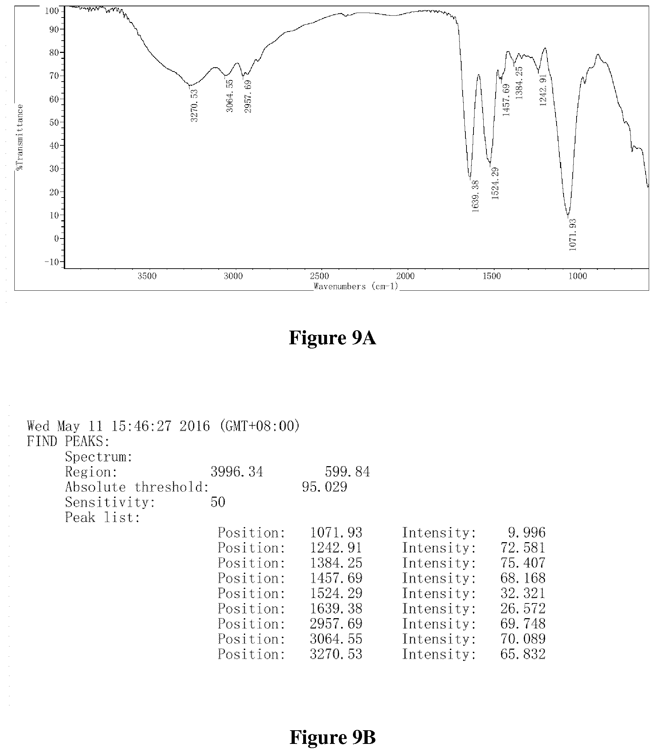

Preferably, said anhydrous crystal A has an infrared absorption spectrum having characteristic bands at 1071.93 cm⁻¹, 1242.91 cm⁻¹, 1384.25 cm⁻¹, 1457.69 cm⁻¹, 1524.29 cm⁻¹, 1639.38 cm⁻¹, 2957.69 cm⁻¹, 3064.55 cm⁻¹ and 3270.53 cm⁻¹ as measured by KBr tableting method; and more preferably, said anhydrous crystal A has an infrared absorption spectrum as measured by KBr tableting method as shown in Figure 9A.

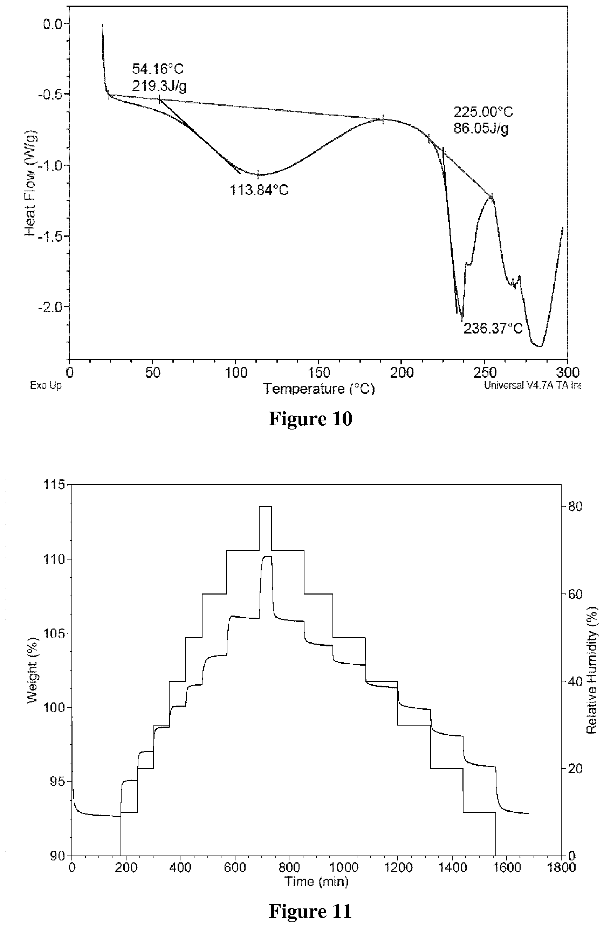

More preferably, said anhydrous crystal A has a melting point of 225.00 °C, and a differential scanning calorimetry pattern as shown in Figure 10.

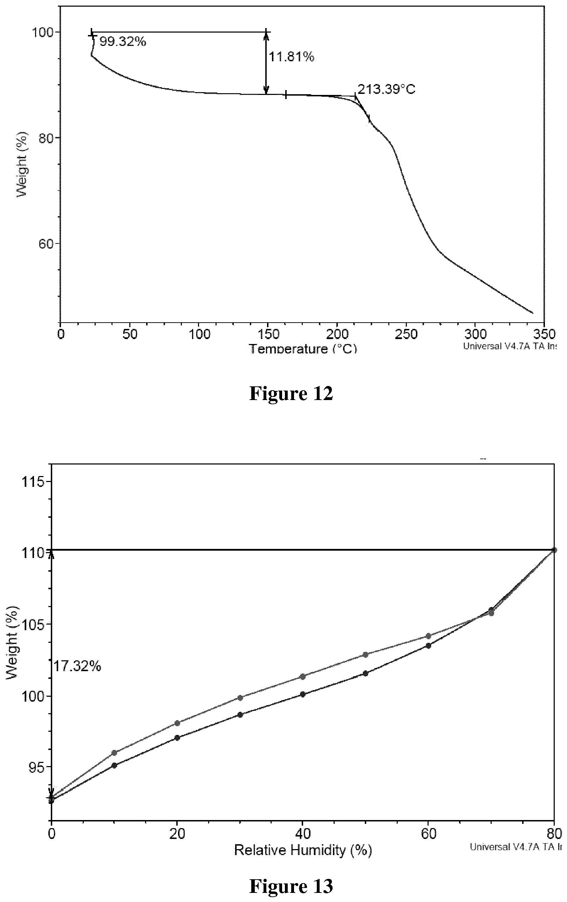

The present inventor conducted a more detailed analysis on anhydrous crystal A, in which said anhydrous crystal A exhibits a dynamic moisture adsorption analysis spectrum as shown in Figure 11, a thermogravimetric analysis spectrum as shown in Figure 12, and an isotherm diagram as shown in Figure 13.

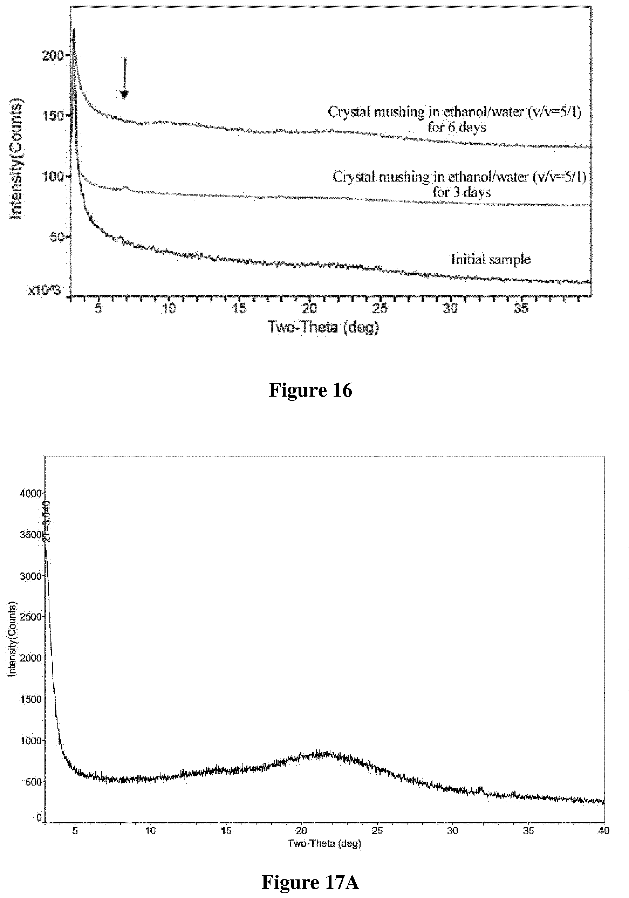

The present invention also provides an anhydrous crystal of polymyxin B2 sulfate, said anhydrous crystal of polymyxin B2 sulfate has an X-ray powder diffraction pattern expressed by 2θ degree using Cu-Ka radiation as shown in Figure 17A.

The present invention also provides a method for preparing an anhydrous crystal of polymyxin B1 sulfate, polymyxin B2 sulfate or a mixture thereof, said method comprises the following steps of:
(1) adding water to polymyxin B1 sulfate, polymyxin B2 sulfate or a mixture thereof to just completely dissolve the solid to obtain a saturated solution;
(2) slowly adding an organic solvent dropwise into said saturated solution, or slowly adding said saturated solution dropwise into an organic solvent at a controlled temperature within the range of 0-60 °C to precipitate a solid; wherein, said organic solvent is selected from one or more of C1-C4 alcohol, C3-C4 ketone, ethyl acetate or butyl acetate; preferably, said C1-C4 alcohol is selected from one or more of methanol, ethanol, isopropanol, n-propanol or n-butanol; and still preferably, said C3-C4 ketone is selected from one or more of acetone or 2-butanone; and
(3) filtering off the solid and drying it under vacuum to obtain an anhydrous crystal of polymyxin B1 sulfate, polymyxin B2 sulfate or a mixture thereof.

In one embodiment of the method for preparing the anhydrous crystal of polymyxin B1 sulfate, polymyxin B2 sulfate or a mixture thereof according to the present invention, in step (1), after adding water to polymyxin B1 sulfate, polymyxin B2 sulfate or a mixture thereof, the solid is just completely dissolved by heating at a temperature below 60 °C.

In another embodiment of the method for preparing the anhydrous crystal of polymyxin B1 sulfate, polymyxin B2 sulfate or a mixture thereof according to the present invention, in step (2), said organic solvent is used in an amount of 0.5-20 volumes in terms of the volume of said saturated solution. The organic solvent can precipitate a solid as well as dissolve impurities, so that the precipitated solid is loose and non-sticky.

In another embodiment of the method for preparing the anhydrous crystal of polymyxin B1 sulfate, polymyxin B2 sulfate or a mixture thereof according to the present invention, in step (2), after the solid is precipitated, stirring is continued for 0-8 hours.

The present invention also provides a method for preparing the anhydrous crystal 1 of polymyxin B1 sulfate, said method comprises the following steps of:
(1) adding water to polymyxin B1 sulfate to just completely dissolve the solid to obtain a saturated solution;
(2) slowly adding an organic solvent dropwise into said saturated solution, or slowly adding said saturated solution dropwise into an organic solvent at a controlled temperature within the range of 0-60°C to precipitate a solid; wherein, said organic solvent is selected from n-butanol, isopropanol, n-propanol, or 2-butanol; and
(3) filtering off the solid and drying it under vacuum to obtain an anhydrous crystal 1 of polymyxin B1 sulfate.

In one embodiment of the method for preparing the anhydrous crystal 1 of polymyxin B1 sulfate according to the present invention, in step (1), after adding water to polymyxin B1 sulfate, the solid is just completely dissolved by heating at a temperature below 60 °C.

In one embodiment of the method for preparing the anhydrous crystal 1 of polymyxin B1 sulfate according to the present invention, in step (2), said organic solvent is used in an amount of 0.5-20 volumes in terms of the volume of said saturated solution.

In one embodiment of the method for preparing the anhydrous crystal 1 of polymyxin B1 sulfate according to the present invention, in step (2), after the solid is precipitated, stirring is continued for 0-8 hours.

The present invention also provides a method for preparing the anhydrous crystal A of polymyxin B1 sulfate, said method comprises the following steps of:
(1) adding water to polymyxin B1 sulfate to just completely dissolve the solid to obtain a saturated solution;
(2) slowly adding an organic solvent dropwise into said saturated solution, or slowly adding said saturated solution dropwise into an organic solvent at a controlled temperature within the range of 0-60 °C to precipitate a solid; wherein, said organic solvent is selected from ethanol, ethanol-n-butanol, n-butanol-isopropanol, methanol, acetone, butanone, or ethanol-ethyl acetate; and
(3) filtering off the solid and drying it under vacuum to obtain an anhydrous crystal A of polymyxin B1 sulfate.

In one embodiment of the method for preparing the anhydrous crystal A of polymyxin B1 sulfate according to the present invention, in step (1), after adding water to polymyxin B1 sulfate, the solid is just completely dissolved by heating at a temperature below 60 °C.

In one embodiment of the method for preparing the anhydrous crystal A of polymyxin B1 sulfate according to the present invention, in step (2), said organic solvent is used in an amount of 0.5-20 volumes in terms of the volume of said saturated solution.

In one embodiment of the method for preparing the anhydrous crystal A of polymyxin B1 sulfate according to the present invention, in step (2), after the solid is precipitated, stirring is continued for 0-8 hours.

Compared with conventional methods, the preparation method of the present invention can obtain an anhydrous crystal of polymyxin B1 sulfate, polymyxin B2 sulfate or a mixture thereof, and the resulting crystal particles are loose and non-sticky, which is particularly advantageous for industrial production of pharmaceuticals. In addition, the preparation method of the present invention can effectively remove impurities in the raw material of the drug, significantly improving the quality of the drug. The crystalline polymyxin B1 sulfate, polymyxin B2 sulfate or a mixture thereof obtained by the method of the present invention is more advantageous for the skilled person to perform formulation processing and efficacy evaluation.

The present invention also provides a pharmaceutical composition, comprising the anhydrous crystal of polymyxin B1 sulfate, polymyxin B2 sulfate or a mixture thereof according to the present invention. The pharmaceutical composition provided by the present invention may also comprise a pharmaceutically acceptable carrier or excipient, and optionally an antibacterial active ingredient other than the anhydrous crystal of polymyxin B1 sulfate, polymyxin B2 sulfate or a mixture thereof.

The present invention also provides the use of the anhydrous crystal of polymyxin B1 sulfate, polymyxin B2 sulfate or a mixture thereof, in particular the anhydrous crystal 1 of polymyxin B1 sulfate, the anhydrous crystal A of polymyxin B1 sulfate or the anhydrous crystal of polymyxin B2 sulfate in the preparation of a medicament for preventing and/or treating a disease caused by bacteria, in particular Gram-negative bacteria. Preferably, said disease is selected from various infections caused by Gram-negative bacteria, in particular *Pseudomonas aeruginosa* and *Escherichia coli*, such as respiratory system infection, peritonitis, bile duct infection, urinary tract infection, burn infection, corneal infection and sepsis, etc..

The present invention also provides a method for preventing and/or treating a disease caused by bacteria, in particular Gram-negative bacteria, comprising administering to a subject a prophylactically and/or therapeutically effective amount of the anhydrous crystal of polymyxin B1 sulfate, polymyxin B2 sulfate or a mixture thereof.

### Brief Description of the Drawings

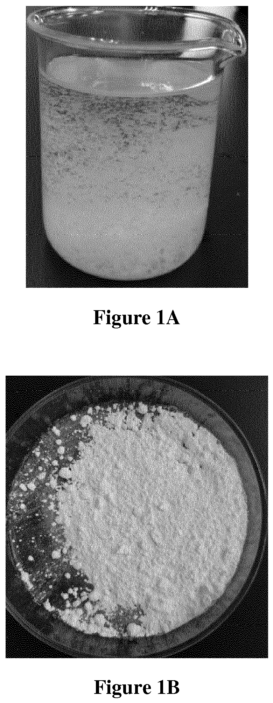

Figure 1A shows the crystal of polymyxin B1 sulfate in an ethanol-n-butanol aqueous solution;
Figure 1B shows a sample of crystal of polymyxin B1 sulfate after drying;
Figure 2A is an XRD pattern of the crystal 1 of polymyxin B1 sulfate of the present invention;
Figure 2B is a peak list of the XRD pattern of the crystal 1 of polymyxin B1 sulfate of the present invention;
Figure 2C is a hot stage XRD pattern of the crystal 1 of polymyxin B1 sulfate of the present invention;
Figure 3A is an IR spectrum of the crystal 1 of polymyxin B1 sulfate of the present invention;
Figure 3B is a peak list of the IR spectrum of the crystal 1 of polymyxin B1 sulfate of the present invention;
Figure 4 is a DSC pattern of the crystal 1 of polymyxin B1 sulfate of the present invention;

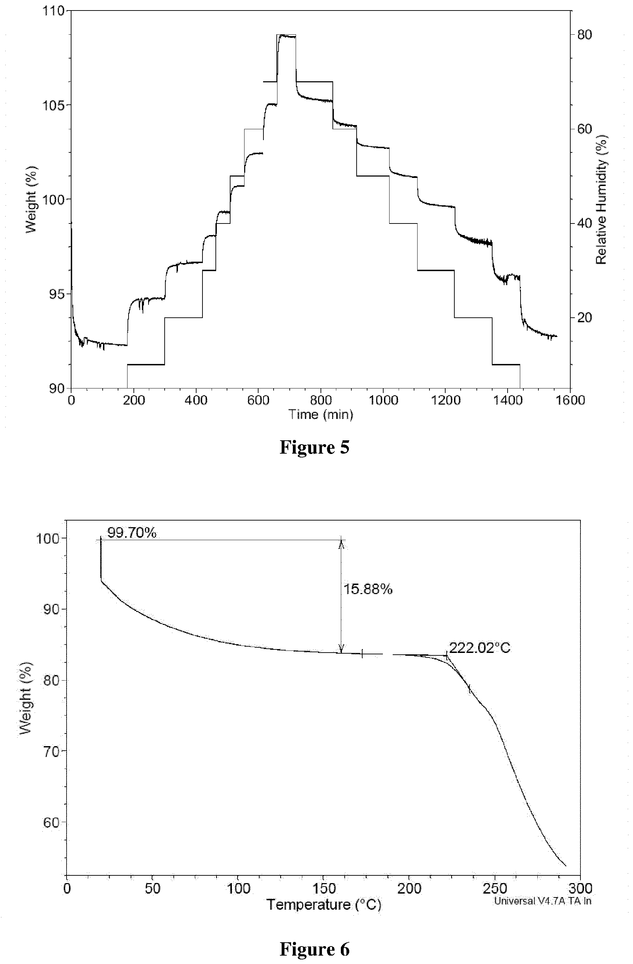

Figure 5 is a DVS pattern of the crystal 1 of polymyxin B1 sulfate of the present invention;
Figure 6 is a TGA spectrum of the crystal 1 of polymyxin B1 sulfate of the present invention;
Figure 7 is an isotherm diagram of the crystal 1 of polymyxin B1 sulfate of the present invention;
Figure 8A is an XRD pattern of the crystal A of polymyxin B1 sulfate of the present invention;

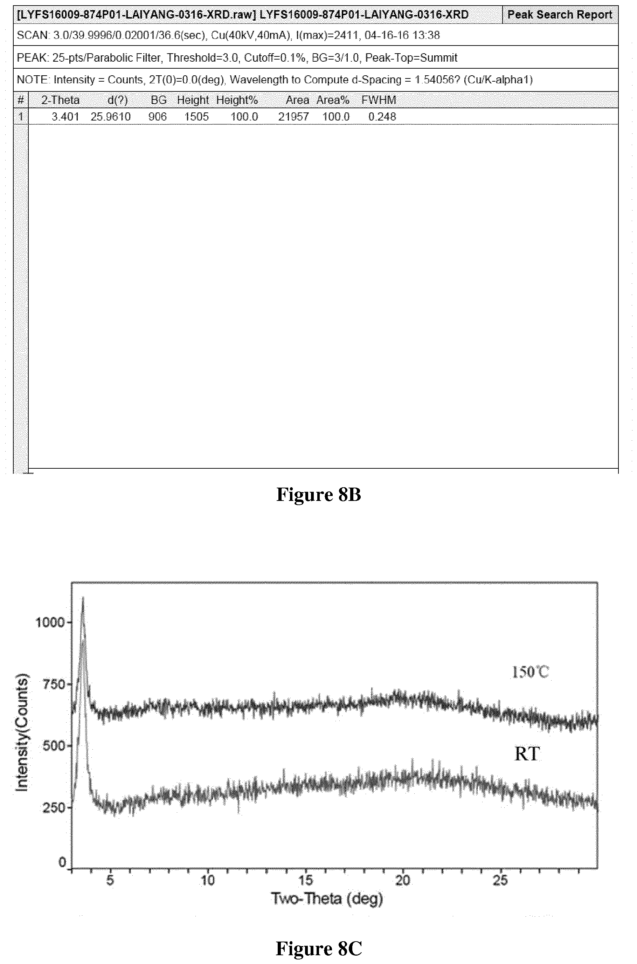

Figure 8B is a peak list of the XRD pattern of the crystal A of polymyxin B1 sulfate of the present invention;
Figure 8C is a hot stage XRD pattern of the crystal A of polymyxin B1 sulfate of the present invention;
Figure 9A is an IR spectrum of the crystal A of polymyxin B1 sulfate of the present invention;
Figure 9B is a peak list of the IR spectrum of the crystal A of polymyxin B1 sulfate of the present invention;
Figure 10 is a DSC pattern of the crystal A of polymyxin B1 sulfate of the present invention;
Figure 11 is a DVS pattern of the crystal A of polymyxin B1 sulfate of the present invention;
Figure 12 is a TGA spectrum of the crystal A of polymyxin B1 sulfate of the present invention;
Figure 13 is an isotherm diagram of the crystal A of polymyxin B1 sulfate of the present invention;

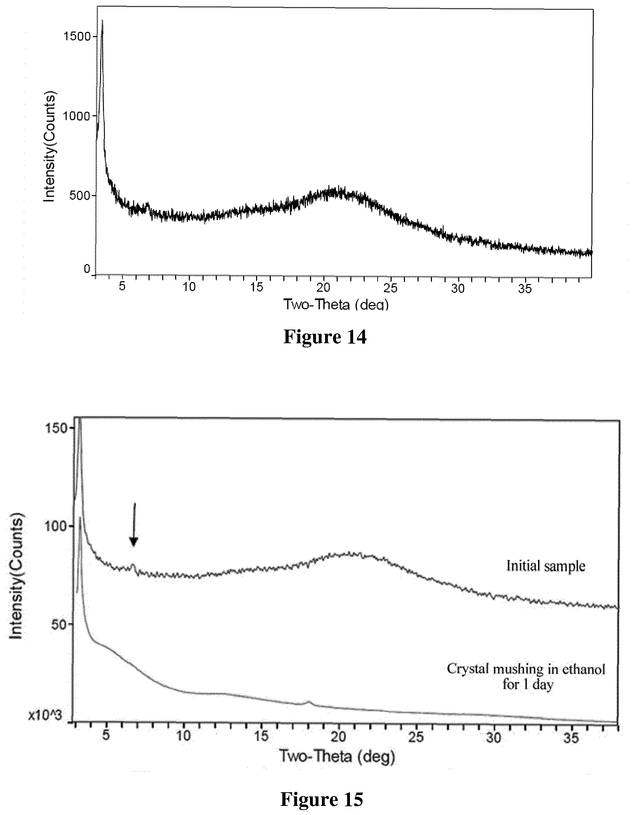

Figure 14 is an XRD pattern of an initial mixed sample of polymyxin B1 sulfate crystal 1 and crystal A in a crystal form competitive experiment of the present invention;
Figure 15 is an XRD pattern of the polymyxin B1 sulfate crystal 1 and crystal A of the present invention in ethanol in a competitive experiment;
Figure 16 is an XRD pattern of the polymyxin B1 sulfate crystal 1 and crystal A of the present invention in ethanol/water mixed solvent (v/v=5/1) in a competitive experiment;
Figure 17A is an XRD pattern of the crystal of polymyxin B2 sulfate of the present invention; and

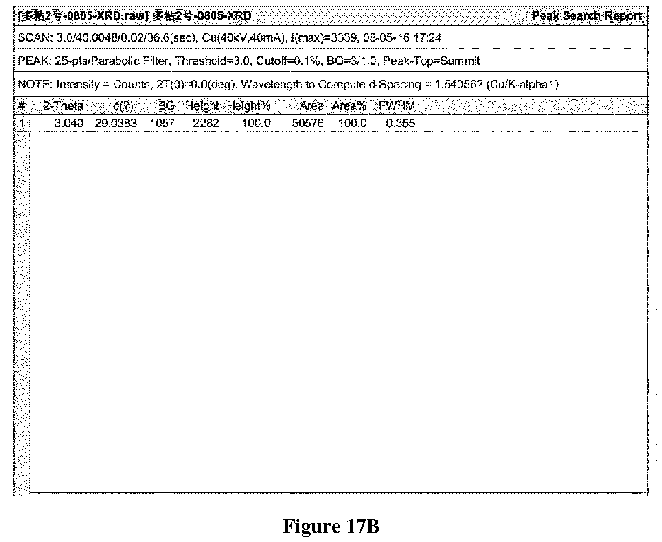

Figure 17B is a peak list of the XRD pattern of the crystal of polymyxin B2 sulfate of the present invention.

### Best Mode for Carrying Out the Invention

The present invention will be further described in conjunction with the accompanying drawings and the embodiments to achieve better illustration of the present invention and to facilitate understanding of the technical solution of the present invention. It is to be understood that the specific examples of the present invention are only intended to illustrate the present invention and are not intended to limit the scope of the present invention.

### Example 1

Ethanol-water system: 100 ml aqueous solution of polymyxin B1 sulfate at a concentration of 50 g/L was taken and stirred at room temperature, to which was added 3 volumes of a 95% (v/v) ethanol aqueous solution to precipitate a polymyxin B1 sulfate crystal. After stirring at a temperature of 0-5 °C for 3-5 h, the mixture was filtered, and dried under vacuum for 8 hours to obtain a crystalline polymyxin B1 sulfate, and the crystal form was crystal form A.

### Example 2

Isopropanol-water: 26 ml aqueous solution of polymyxin B1 sulfate and polymyxin B2 sulfate at a concentration of 50 g/L was taken, and slowly added dropwise to 260 ml (10 volumes) isopropanol under stirring. The temperature of isopropanol was always controlled within the range of 5-10 °C. The crystals of polymyxin B1 sulfate and polymyxin B2 sulfate precipitated during the dropwise adding gradually increased, which was in a uniform dispersion state without adhesion. Stirring was continued for 1 hour, the mixture was filtered to obtain a solid, which was dried under vacuum for 10 hours to obtain a crystal powder of polymyxin B1 sulfate and polymyxin B2 sulfate (1.23g), crystal yield: 94.6%. The crystal form of polymyxin B1 sulfate was crystal form 1.

### Example 3

N-butanol-water: 13.5 g polymyxin B2 sulfate was dissolved in 150 ml pure water, and slowly added dropwise to 1500 ml (10 volumes) n-butanol under stirring with the temperature being always controlled within the range of 25-30 °C. The crystal of polymyxin B2 sulfate precipitated during the dropwise adding gradually increased, which was in a uniform dispersion state without adhesion and had a good granularity. After the dropwise addition of the aqueous solution of polymyxin B2 sulfate was completed, stirring was continued for 30 min, the mixture was filtered, and the filtered solid was dried under vacuum for 20 hours to obtain a crystal powder of polymyxin B2 sulfate (13.2 g), crystal yield: 97.8%.

From the XRD pattern of polymyxin B2 sulfate and polymyxin B1 sulfate, it can be seen that in the XRD pattern of polymyxin B2 sulfate, a weak small peak appears at 2θ = 31.8°.

### Example 4

Ethanol-n-butanol-water: 100 ml aqueous solution of polymyxin B1 sulfate at a concentration of 50 g/L was taken and slowly added dropwise to 1000 ml (10 volumes) ethanol (500 ml)+n-butanol (500 ml) under stirring, and the temperature during the dropwise adding was always controlled within the range of 0-5°C. The crystal of polymyxin B1 sulfate precipitated during the dropwise adding gradually increased, which was in a uniform dispersion state and had a good granularity. After the dropwise addition of the aqueous solution of polymyxin B1sulfate was completed, stirring was continued for 2 hours, a solid was filtered off and was dried under vacuum for 5 hours to obtain a crystal powder of polymyxin B1 sulfate (4.6 g), crystal yield: 92.0%. The crystal form was crystal form A.

### Example 5

Ethanol-isopropanol-water: 10 ml aqueous solution of polymyxin B1 sulfate and polymyxin B2 sulfate at a concentration of 50 g/L was taken and slowly added dropwise to 100 ml (10 volumes) ethanol (50 ml) +isopropanol (50 ml) under stirring, and the temperature during the dropwise adding was always controlled within the range of 15-20 °C. The crystal of polymyxin B1 sulfate and polymyxin B2 sulfate precipitated during the dropwise adding gradually increased, which was in a uniform dispersion state and had a granularity, with solid-liquid separation being quickly achieved after standing (30 min). After the dropwise addition of the aqueous solution of polymyxin B1 sulfate and polymyxin B2 sulfate was completed, stirring was continued for 30 min, a solid was filtered off and was dried under vacuum for 15 hours to obtain a crystal powder of polymyxin B1 sulfate and polymyxin B2 sulfate (0.43 g), crystal yield: 86.0%. The crystal form was crystal form A.

### Example 6

N-butanol-isopropanol-water: 50 ml aqueous solution of polymyxin B1 sulfate at a concentration of 50 g/L was taken and slowly added dropwise to 250 ml (5BV) n-butanol (125 ml)+isopropanol (125 ml) under stirring at a controlled temperature within the range of 25-30 °C. After the crystal precipitated, the remaining solution was added completely to obtain a crystal, which was in a uniform dispersion state and had a good granularity, with solid-liquid separation being quickly achieved after standing (30min). After the dropwise addition of the aqueous solution of polymyxin B1 sulfate was completed, stirring was continued for 60 min, a solid was filtered off and was dried under vacuum for 8 hours to obtain a crystal powder of polymyxin B1 sulfate (2.3 g), crystal yield: 92.0%. The crystal form was crystal form 1.

### Example 7

Characterization and analysis of crystal form 1 and crystal form A of polymyxin B1 sulfate

**Crystal form 1 of polymyxin B1 sulfate**

| Characterization mode | Characterization results and analysis |
|---|---|
| XRD | Weak crystalline state, numbered as crystal form 1 |
| Hot stage XRD | The crystal form has not changed when heating up to 170 °C |
| DSC/TGA | DSC pattern shows that the sample of crystal form 1 has a broad endothermic peak at 50-200 °C, which is due to the removal of the surface solvent, and the melting point is 226.97 °C, accompanied by decomposition; |
| | TGA spectrum shows a 16% slow weight loss before 150 °C, which was confirmed to be a surface solvent in conjunction with hot stage XRD, and the decomposition temperature was 222 °C. |
| DVS | Weight change between 0%RH-80%RH is about 15.9%, with an extremely strong hygroscopicity. |
| FTIR | The absorption peaks in detail are shown in Figure 3A. |
| HPLC | The purity is 98.43%. |
| | |

| Description of crystal form | |
|---|---|
| Property of crystal form | Anhydrate |
| Preparation | It can be obtained by crystal mushing a crystal form A of polymyxin B1 sulfate in a mixed solution of isopropyl alcohol and water or a mixed solution of n-propanol and water, or it can be obtained by crystal mushing a crystal form A of polymyxin B1 sulfate at a high temperature in butanol or 2-butanol solution. |
| Typical preparation method | 50 mg crystal form A of polymyxin B1 sulfate was taken and added into 1 mL mixed solvent of isopropanol/water=4/1 to crystal mush at room temperature for more than 5 days and centrifuge. |
| Stability evaluation | Unstable, the crystal form changed to crystal form A when placed in a dry state at room temperature for 3 days. |

**Crystal form A of polymyxin B1 sulfate**

| Characterization mode | Characterization results and analysis |
|---|---|
| XRD | Weak crystalline state, numbered as crystal form A |
| Hot stage XRD | The crystal form has not changed when heating up to 150 °C and keeping for 5 min. |
| DSC/TGA | DSC pattern shows that the sample of crystal form A has a broad endothermic peak before 200 °C, which is due to the removal of the surface solvent, and the melting point is 225.00 °C, accompanied by decomposition; |
| | TGA spectrum shows an about 11.8% slow weight loss before 150 °C, which was confirmed to be a surface solvent in conjunction with hot stage XRD, and the decomposition temperature was 213 °C. |
| DVS/Isothermal adsorption diagram | Weight change between 0%RH-80%RH is about 17.3%, with an extremely strong hygroscopicity. |
| FT-IR | The absorption peaks in detail are shown in Figure 9A. |
| HPLC | The purity is 98.43%. |

### Example 8

A competitive experiment between crystal form 1 and crystal form A of polymyxin sulfate was carried out to investigate crystal form stability of the two crystal forms in the solvent, water and ethanol, which are commonly used in the preparation, and to evaluate the conversion of the two crystal forms, in order to provide reference for subsequent preparation operations such as granulation. In this experiment, a mixed solvent of ethanol and ethanol/water=5/1 (v/v) was used as the solvent for the competitive experiment, to confirm the most stable crystal form at room temperature in the corresponding solvent in combination with crystal mush test at room temperature.

### Crystal form competitive experiment

1. Equal amounts of samples of crystal form 1 and form A were taken and mixed uniformly to obtain an initial mixed sample, which was used for XRD detection (Figure 14).
2. The uniformly-mixed sample was divided into two portions, to one of which was added 200 µL ethanol, and to another of which was added 300 µL mixed solvent of ethanol/water=5/1(v/v), to form a suspension. The suspension was stirred at room temperature, and centrifuged and sampled at different times for XRD detection. The results showed that after crystal mushing in ethanol at room temperature for one day, the crystal form converted into crystal form A (Figure 15), and after crystal mushing in ethanol/water at room temperature for six days, the crystal form converted into crystal form A (Figure 16).

According to the results of this competitive experiment, it was confirmed that among the two crystal forms of polymyxin B1 sulfate, the most stable crystal form in ethanol and in a mixed solvent of ethanol/water at room temperature is crystal form A.

According to the results of crystal mush experiment at room temperature, it was confirmed that the most stable crystal form of polymyxin B1 sulfate in ethanol and in a mixed solvent of ethanol/water at room temperature is crystal form A.

Based on the above results, when polymyxin B1 sulfate is handled at room temperature, polymyxin B1 sulfate can be stably present as the crystal form A using ethanol or a mixed solvent of ethanol/water.

## Claims

1. An anhydrous crystal 1 of polymyxin B1 sulfate, **characterized in that**, said anhydrous crystal 1 has an X-ray powder diffraction pattern having diffraction peaks at 3.396, 4.895 and 6.903 expressed by 2θ degree using Cu-Ka radiation; and preferably, said crystal 1 has an X-ray powder diffraction pattern expressed by 2θ degree using Cu-Ka radiation as shown in Figure 2A;
preferably, said anhydrous crystal 1 has an infrared absorption spectrum having characteristic bands at 1071.93 cm⁻¹, 1242.91 cm⁻¹, 1384.25 cm⁻¹, 1457.69 cm⁻¹, 1524.29 cm⁻¹, 1639.38 cm⁻¹, 2957.69 cm⁻¹, 3064.55 cm⁻¹ and 3270.53 cm⁻¹ as measured by KBr tableting method; and more preferably, said anhydrous crystal 1 has an infrared absorption spectrum as measured by KBr tableting method as shown in Figure 3A; and
more preferably, said anhydrous crystal 1 has a melting point of 226.97 °C, and has a differential scanning calorimetry pattern as shown in Figure 4.

2. An anhydrous crystal A of polymyxin B1 sulfate, **characterized in that**, said anhydrous crystal A has an X-ray powder diffraction pattern having a diffraction peak at 3.401 expressed by 2θ degree using Cu-Ka radiation; and preferably, said anhydrous crystal A has an X-ray powder diffraction pattern expressed by 2θ degree using Cu-Ka radiation as shown in Figure 8A;
preferably, said anhydrous crystal A has an infrared absorption spectrum having characteristic bands at 1071.93 cm⁻¹, 1242.91 cm⁻¹, 1384.25 cm⁻¹, 1457.69 cm⁻¹, 1524.29 cm⁻¹, 1639.38 cm⁻¹, 2957.69 cm⁻¹, 3064.55 cm⁻¹ and 3270.53 cm⁻¹ as measured by KBr tableting method; and more preferably, said anhydrous crystal A has an infrared absorption spectrum as measured by KBr tableting method as shown in Figure 9A; and
more preferably, said anhydrous crystal A has a melting point of 225.00 °C, and a differential scanning calorimetry pattern as shown in Figure 10.

3. An anhydrous crystal of polymyxin B2 sulfate, **characterized in that**, said anhydrous crystal of polymyxin B2 sulfate has an X-ray powder diffraction pattern expressed by 2θ degree using Cu-Ka radiation as shown in Figure 17A.

4. A method for preparing an anhydrous crystal of polymyxin B1 sulfate, polymyxin B2 sulfate or a mixture thereof, said method comprises the following steps of:
(1) adding water to polymyxin B1 sulfate, polymyxin B2 sulfate or a mixture thereof to just completely dissolve the solid to obtain a saturated solution;
(2) slowly adding an organic solvent dropwise into said saturated solution, or slowly adding said saturated solution dropwise into an organic solvent at a controlled temperature within the range of 0-60 °C to precipitate a solid; wherein, said organic solvent is selected from one or more of C1-C4 alcohol, C3-C4 ketone, ethyl acetate or butyl acetate; preferably, said C1-C4 alcohol is selected from one or more of methanol, ethanol, isopropanol, n-propanol or n-butanol; and still preferably, said C3-C4 ketone is selected from one or more of acetone or 2-butanone; and
(3) filtering off the solid and drying it under vacuum to obtain an anhydrous crystal of polymyxin B1 sulfate, polymyxin B2 sulfate or a mixture thereof.

5. A method for preparing the anhydrous crystal 1 of polymyxin B1 sulfate according to claim 1, said method comprises the following steps of:
(1) adding water to polymyxin B1 sulfate to just completely dissolve the solid to obtain a saturated solution;
(2) slowly adding an organic solvent dropwise into said saturated solution, or slowly adding said saturated solution dropwise into an organic solvent at a controlled temperature within the range of 0-60 °C to precipitate a solid; wherein, said organic solvent is selected from n-butanol, isopropanol, n-propanol, or 2-butanol; and
(3) filtering off the solid and drying it under vacuum to obtain an anhydrous crystal 1 of polymyxin B1 sulfate.

6. A method for preparing the anhydrous crystal A of polymyxin B1 sulfate according to claim 2, said method comprises the following steps of:
(1) adding water to polymyxin B1 sulfate to just completely dissolve the solid to obtain a saturated solution;
(2) slowly adding an organic solvent dropwise into said saturated solution, or slowly adding said saturated solution dropwise into an organic solvent at a controlled temperature within the range of 0-60 °C to precipitate a solid; wherein, said organic solvent is selected from ethanol, ethanol-n-butanol, n-butanol-isopropanol, methanol, acetone, butanone, or ethanol-ethyl acetate; and
(3) filtering off the solid and drying it under vacuum to obtain an anhydrous crystal A of polymyxin B1 sulfate.

7. The method according to any one of claims 4 to 6, **characterized in that**, in step (1), after adding water to polymyxin B1 sulfate, polymyxin B2 sulfate or a mixture thereof, the solid is just completely dissolved by heating it at a temperature below 60 °C;
preferably, in step (2), said organic solvent is used in an amount of 0.5-20 volumes in terms of the volume of said saturated solution; and
more preferably, in step (2), after the solid is precipitated, stirring is continued for 0-8 hours.

8. A pharmaceutical composition comprising the anhydrous crystal 1 of polymyxin B1 sulfate according to claim 1, the anhydrous crystal A of polymyxin B1 sulfate according to claim 2, the anhydrous crystal of polymyxin B2 sulfate according to claim 3, or the anhydrous crystal of polymyxin B1 sulfate, polymyxin B2 sulfate or a mixture thereof prepared according to the method according to any one of claims 4 to 7.

9. The pharmaceutical composition according to claim 8, comprising a pharmaceutically acceptable carrier or excipient, and optionally an antibacterial active ingredient other than the anhydrous crystal of polymyxin B1 sulfate, polymyxin B2 sulfate or a mixture thereof.

10. Use of the anhydrous crystal 1 of polymyxin B1 sulfate according to claim 1, the anhydrous crystal A of polymyxin B1 sulfate according to claim 2, the anhydrous crystal of polymyxin B2 sulfate according to claim 3, or the anhydrous crystal of polymyxin B1 sulfate, polymyxin B2 sulfate or a mixture thereof prepared according to the method according to any one of claims 4 to 7 in the preparation of a medicament for preventing and/or treating a disease caused by bacteria, in particular Gram-negative bacteria.

11. The use according to claim 10, wherein said disease is selected from various infections caused by Gram-negative bacteria, in particular *Pseudomonas aeruginosa* and *Escherichia coli*; and preferably, said disease is respiratory system infection, peritonitis, bile duct infection, urinary tract infection, burn infection, corneal infection and sepsis.

12. A method for preventing and/or treating a disease caused by bacteria, in particular Gram-negative bacteria, comprising administering to a subject a prophylactically and/or therapeutically effective amount of the anhydrous crystal 1 of polymyxin B1 sulfate according to claim 1, the anhydrous crystal A of polymyxin B1 sulfate according to claim 2, the anhydrous crystal of polymyxin B2 sulfate according to claim 3, or the anhydrous crystal of polymyxin B1 sulfate, polymyxin B2 sulfate or a mixture thereof prepared according to the method according to any one of claims 4 to 7
